Europäisches Patentamt

European Patent Office

Office européen des brevets

(19)

(11) Publication number : **0 653 203 A1**

# (12) EUROPEAN PATENT APPLICATION

(21) Application number : **94305785.1**

(22) Date of filing : **04.08.94**

(51) Int. Cl.$^6$ : **A61K 7/38,** C07F 7/00

(30) Priority : **10.08.93 US 105312**

(43) Date of publication of application :
**17.05.95 Bulletin 95/20**

(84) Designated Contracting States :
**DE ES FR GB GR IT SE**

(71) Applicant : **Bristol-Myers Squibb Company**
**345 Park Avenue**
**New York, N.Y. 10154 (US)**

(72) Inventor : **Rosenberg, Allan H.**
**129 Irving Avenue**
**South Orange, NJ 07079 (US)**
Inventor : **Markarian, Herand M.**
**160 Waters Edge**
**Congers, NY 10920 (US)**
Inventor : **Onyszkewycz, Maria**
**387 16th Avenue**
**Irvington, NY 07111 (US)**
Inventor : **Tencza, Thomas**
**31 Wagner Avenue**
**Wallington, NJ 07057 (US)**

(74) Representative : **Cropp, John Anthony David et al**
**MATHYS & SQUIRE**
**100 Grays Inn Road**
**London, WC1X 8AL (GB)**

(54) **Novel zirconium salts and their synthesis.**

(57) An improved process for the preparation of aluminum/zirconium/glycine complexes, which is particularly suited to the production of novel ZAG salt complexes having enhanced antiperspirant efficacy, comprises

forming an aqueous admixture of a zirconium starting material and glycine at ambient temperature ;

combining said zirconium/glycine aqueous mixture with an aluminum chlorohydrate starting material ; and

rapidly drying the resultant aluminum chlorohydrate/glycine/zirconium mixture. The aluminum/zirconium/glycine complexes produced thereby and antiperspirant compositions containing them are also disclosed.

EP 0 653 203 A1

## FIELD OF INVENTION

The present invention concerns antiperspirant salts containing an aluminum species, a zirconium species and an amino acid that have superior efficacy in view of the low molecular weight distribution of the zirconium species, which molecular weight distribution is controlled by proper selection and regulation of the processing conditions used in preparing the antiperspirant salt. The present invention is especially related to a high efficacy antiperspirant salt containing the low molecular weight zirconium species of the present invention, in admixture with an activated aluminum chloro species and an amino acid.

## BACKGROUND OF THE INVENTION

The preparation of aluminum- and zirconium-based antiperspirant materials generally involves combining compounds of these metals with an amino acid under suitable conditions. Such materials are generally referred to as ZAG complexes and have often been shown to be chemically distinct species having antiperspirant efficacy, i.e., the inhibition of perspiration when applied to human skin, especially the axilla.

In recent years various workers have attempted to enhance efficacy of ZAG salts by preparing such salts that contain an activated aluminum chlorohydrex polymeric salt (ACH) in which a large amount of the Al Kd 0.4 species ($\geqq$ 50%) is present. These so-called activated ZAG salts were thought to be superior to normal ZAG salts due to the increased Al Kd 0.4 content as is the case for activated ACH compared to normal ACH. We have found, however, that activated ZAG salts are not necessarily more efficacious than normal ZAG salts and in fact can be less efficacious than normal ZAG salts.

In U.S. Patent 4,775,528 to Callaghan et al, an antiperspirant composition is produced by heating a 2 to 18% aqueous solution of aluminum chlorhydroxide to at least 50°C, mixing zirconyl hydroxychloride with the solution before, during or after heating, continuing heating of the aqueous mixture until prescribed characterizations are achieved, and thereafter rapidly drying the heated aqueous mixture to obtain a solid material having an Al:Zr atomic ratio of from 6:1 to 1:1 and also having peak 4 and peak 3 Al chromatograph peaks, the ratio of peak 4 to peak 3 being at least 2:1. Patentees specify in Claim 1 that the zirconyl hydroxychloride be mixed with the aluminum chlorhydroxide solution before the solution loses all of its water, i.e., "before completion of ... drying".

The teachings of Callaghan, et al are directed to improvements to be achieved via optimizing the aluminum chemistry in the production of a ZAG salt complex, without any regard for the effects of the zirconium chemistry on the resultant product.

Likewise, U.S. Patent 4,871,525 to Giovanniello, et al. also teaches a method to activate ZAG complexes by essentially enriching the Al Kd 0.4 content.

The above patents do not mention anything about how Zr distributions affect efficacy and do not provide any data showing that Zr distributions are different depending upon Zr starting materials and synthesis procedures.

Contrary to applicant's findings as taught herein, the aforementioned patents encourage the use of heat to prepare the Al and Zr components and in mixing the components. The importance of adding the amino acid component immediately to the Zr component is not addressed.

In fact, applicant knows of no prior art references which speak to the advantages to be afforded by suitably controlling the parameters which effect the zirconium chemistry, primarily it is believed because no such effects were heretofore recognized nor the positive advantages to be achieved by properly controlling the relevant parameters clearly understood.

## SUMMARY OF INVENTION

The present invention is directed to aluminum/zirconium/amino acid complexes which have enhanced antiperspirant activity. The improved characteristics of the antiperspirant (A/P) active complex of the present invention result from the unique process parameters used to manufacture the complex, which complex forms by admixture of an aluminum chlorohydrex polymeric salt, a zirconium species and an amino acid. An activated aluminum chlorohydrex salt (ACH) is especially preferred. The use of glycine and alanine are preferred as the amino acid. Glycine is especially preferred.

Thus, the active complex of the present invention preferably comprises an admixture of an activated aluminum chlorohydrex polymeric salt, a zirconium species and glycine, the admixing of which causes interactions that result in the final preferred complex.

Although such components have been mixed in the prior art in order to provide an antiperspirant complex, the processing parameters disclosed in the present invention result in the new complex possessing superior antiperspirant characteristics. Accordingly, the invention further concerns a method of making the improved antiperspirant active complex described herein.

Further, the present invention is also directed to improved antiperspirant compositions comprising, as an active ingredient, the antiperspirant active complex taught herein.

## DESCRIPTION OF THE FIGURES

Figures 1a and 1b are graphical representations of typical aluminum polymer distributions in normal ZAG and activated ZAG complexes.

Figure 2 is a graphical representation of Zr distributions for a known normal ZAG Salt and known activated ZAG salts all of which were obtained from Summit Chemical (formerly Dow-Corning).

Figure 3 is a graphical representation of Zr distributions for two known activated ZAG salts obtained from Reheis.

Figure 4 is a graphical representation of Zr distributions for two known Westwood activated ZAGS, for an experimental activated ZAG salt developed by the assignee of applicant of the present invention (designated BM-P I), and for the activated ZAG present in a commercially available consumer product.

Figure 5 is a graphical representation of the Zr distributions found in various commercially available Zr starting materials.

Figure 6 is a graphical representation of the effect of heating on ZrO(OH)Cl solutions.

Figure 7 is a graphical representation of the effect of glycine on Zr polymerization.

Figure 8 is another graphical representation of the effect of glycine on Zr polymerization.

Figure 9 is a graphical representation of the effect of contact time on Zr distributions for an activated ACH -ZrOCl$_2$ -glycine salt.

Figure 10 is a graphical representation of Zr distributions for various activated ZAG salts made in accordance with the present invention, and a control.

Figure 11 is a graphical representation of Zr distributions for BM-P I, ZAG salt of the present invention, and a control extracted from solid stick deodorants.

## DETAILED DESCRIPTION OF THE INVENTION

It has been found that one of the most important considerations in achieving the improved antiperspirant active complex of the present invention is to maintain the molecular weight of the zirconium polymer as low as possible in the final aluminum/zirconium/glycine complex. It should be understood that the zirconium polymer has a molecular weight distribution and that the zirconium polymer molecular weight referred to herein is not a discrete value but rather a weight average value. It should also be understood that, while the discussion of the results provided herein is directed to the use of glycine, as the most preferred amino acid component, it will be understood by those skilled this art that other less preferably amino acids, such as for example alanine, may also be utilized to advantage.

It has also now been found that the desirable low Zr polymer molecular weight is achieved by using the following combination of processing parameters:

1) In the absence of heat, preferably at room temperature, admixing in an aqueous system, the glycine with the zirconium species starting material prior to admixture with the aluminum polymeric salt;

2) Avoiding subjecting the resulting aqueous solution containing the Zr -glycine component to heating, and

3) Rapidly intermixing the aluminum polymer solution and the zirconium/glycine solution and following intermixing rapidly spray drying the composite solution.

The meanings of the terms "absence of heat", "avoiding heating", "rapidly intermixing" and "rapidly drying" will be understood by one skilled in this art and are further explained herein below in the context of the discussion of processing parameters.

As noted above, the key to achieving the improved antiperspirant active complex of the present invention is to ensure that the molecular weight of the zirconium polymer is maintained as low as possible in the final aluminum/ zirconium/glycine complex. Most preferably, the molecular weight of the zirconium polymer in the resultant complex will be as near as possible to the molecular weight of the zirconium starting raw material. The zirconium species starting material employed in the manufacture of the ZAG antiperspirant active of the present invention does interact with the ACH and the glycine during the preparation of the complex. For this reason the mixture cannot be regarded as a mere aggregation of chemicals.

Several unique aspects of the process of the present invention aid in keeping the molecular weight of the zirconium polymer as low as possible. First, glycine is admixed with the zirconium polymer prior to admixture with the aluminum polymeric salt. That is, glycine has been found to be an inhibitor to the further polymerization

3

of the zirconium starting material. Secondly, contrary to prior art teachings, neither the zirconium/glycine solution (or premixes if employed) nor the aluminum/zirconium/glycine solution is subjected to heat, since heat has been found to further polymerize the zirconium. Lastly, the aluminum polymer solution and the zirconium/glycine solution are intermixed as rapidly as possible, and then are spray-dried as quickly as possible, after their admixture. Thus, a continuous or batch/continuous process is generally preferred to accomplish this objective.

The ZAG salts produced in accordance with the present invention can be effectively used in non-aqueous delivery systems such as, for example, in sticks, gels, aerosols and suspension roll-ons or pads. A variety of vehicles or carriers used in delivery systems for anti-perspirant compositions are well known to those skilled in this art. For a discussion of those carriers which may be effectively employed in the present invention applicants' have reference to U.S. Patent Nos. 4,944,937 to McCall, 4,511,554 to Geria, et al, 4,425,328 to Nabial, 4,346,079 to Roehl, and 3,949,066 to Clark, the teachings of which are incorporated herein by reference.

Using improved analytical techniques it has now been discovered that Zr polymer distributions present in ZAG salts play a major role in determining salt efficacy and may in fact be more important than Al polymer distributions with respect to salt antiperspirant efficacy.

As noted hereinabove, it has been found that lower molecular weight Zr polymer distributions enhance salt antiperspirant efficacy while higher molecular weight Zr distributions decrease salt efficacy. Thus, the apparent anomaly of the fact that some activated ZAG complexes exhibit equal or lesser efficacy than normal ZAG complexes is easily explained by comparing not only Al Kd 0.4 content but also Zr distributions.

Using the above structure-efficacy results, activated ZAG salts having large Al Kd 0.4 content and relatively low molecular weight Zr distributions have been synthesized.

Therefore, one important contribution of the present invention lies in the discovery of the effect of Zr distribution on ZAG complex antiperspirant activity. Another, lies in the discovery of synthesis procedures which can be used to control both the Al and Zr distributions in the resultant ZAG complex products in order to effect enhanced antiperspirant characteristics.

Method of Preparation

The ZAG salts of the present invention are preferentially prepared by adding a Zr-glycine component $(ZrO(OH)Cl$ -glycine, $ZrOCl_2$ -glycine or a combination of both) to an ACH component, preferably an activated ACH component, and spray drying to the solid ZAG powder. Three examples are:

ACH component + $ZrOCl_2$/glycine

ACH component + $ZrO(OH)Cl - ZrOCl_2$/glycine

ACH component + $ZrO(OH)Cl$/glycine

The critical synthesis parameters necessary to obtain high Al Kd 0.4 content and low molecular weight Zr distributions in the ZAG complex are as follows:

1) The Al component must have Al Kd 0.4 content $\geqq 50\%$

2) The Zr - glycine component must be prepared without the application of heat beyond that required to maintain ambient temperature.

3) The Al and Zr - glycine components must be mixed without the application of heat.

4) The Zr starting material most preferably has the lowest available molecular weight Zr distribution (presently Magnesium Elektron Inc.(MEI) provides Zr starting materials with the lowest molecular weight Zr distributions).

5) The glycine is to be added to the Zr component before mixing with the Al component.

6) Short residence times are to be maintained for the final ZAG reaction solutions before spray drying. A flow system in which the Al and the Zr -glycine components are mixed in a tube leading to the spray dryer is preferred.

It should be understood that the present invention is not limited to ZAG complexes in which the Al component is limited to Al Kd 0.4 content above 50%, as the novel Zr glycine component of the present invention will increase the efficacy of the final ZAG complex in any event.

It has been found that adhering to the aforementioned process parameters will produce the desired Al and Zr distributions. Adverse effects on the metal distribution (i.e. decreasing Al Kd 0.4 content and increasing molecular weight Zr distribution) are observed when heat, the absence of glycine and long residence time of the final ZAG solution are used to prepare ZAG salts.

It is generally expected that the utilization of the process parameters of the present invention can be employed to prepare any stoichiometric combination of Al and Zr -glycine compositions. To applicant's knowledge, the activated ZAG complexes prepared in accordance with the aforementioned process parameters exhibit the lowest molecular weight Zr distribution as compared to other commercially available activated ZAG salts.

The chemistry of antiperspirant salts is quite complex due to the fact that the salts are not homogeneous but consist of polydispersed charged metal (cationic) polymers. In the past ten years, significant advances have been made in elucidating the polymer distributions present in aluminum A/P salts. These discoveries were instrumental in the development of the commercial syntheses of activated aluminum chlorohydrate (ACH) salts such as Reheis RE101, a commercially available material used in formulating commercially sold consumer products. The enhanced efficacy of activated ACH salts as compared to normal ACH salts is generally attributed to the higher concentration of the Al Kd 0.4 polymer species present in the activated salts.

Zirconium-aluminum-glycine (ZAG) salts are in turn generally more efficacious than aluminum salts (including activated ACH) and are used in several commercial consumer suspension roll-on and stick products. Commercial salt suppliers and various consumer product manufacturers have some knowledge of the aluminum chemistry occurring in ZAG systems but none, to the inventors knowledge, and based on the published literature, have an understanding of the effects of the zirconium chemistry occurring within ZAG complex systems. This state of affairs is primarily due to the difficulties inherent in characterizing zirconium polymers. A variety of workers have attempted to improve the efficacy of ZAG salts by producing salts containing large amounts of the aluminum Kd 0.4 polymer, as was previously done for aluminum salts. These salts are referred to as activated ZAG salts. As noted previously, others have issued US patents for activated ZAG salts based upon the enrichment of the Al Kd 0.4 species. Unfortunately, the lack of a precise knowledge of the zirconium chemistry involved can result in situations where so-called "activated" ZAG salts are equal to or less efficacious than normal ZAG salts. This anomaly is believed to be attributable to differences in the polymer distributions of the zirconium polymer present in these ZAG salts, which differences appear to be more important to salt efficacy than the aluminum polymer distributions.

In recent years the applicant here has made substantial gains in understanding the workings of zirconium chemistry in ZAG salts. Using several experimental techniques such as Gel Permeation Chromatograph (GPC), ICP Spectroscopy, Ultracentrifiguration (UC) and NMR Spectroscopy, the applicant has now been able to separate, identify and quantify the Zr polymer distributions present in various ZAG salts. Correlating this information with clinical efficacy studies verifies that ZAG salt complexes with zirconium distributions having predominately lower molecular weight species show enhanced A/P efficacy as compared with ZAG salt complexes with zirconium distributions having predominately higher molecular weight species.

With the above structure - efficacy correlations in mind highly efficacious activated ZAG salts have now been synthesized using commercial processing techniques. These salts proved to be significantly more efficacious than W-370, the ZAG salt commercially available from Summit Chemical, which is used in currently available consumer antiperspirant products.

ANALYTICAL CONSIDERATIONS

Gel Permeation Chromatography (GPC) - Inductively - Coupled Plasma Spectroscopy (ICP)

GPC - ICP analysis has been the primary technique used for characterizing ZAG salts in terms of separating, detecting and measuring aluminum and zirconium polymers present in the system. The GPC column separates species in ZAG systems as a function of molecular size and/or molecular weight with the larger molecules eluting off the column first, followed by the smaller molecules. GPC resins are chosen to optimize separation for a molecular weight range appropriate for the system being analyzed. Separated species are detected by means of a refractive index (RI) detector connected to the column outlet. Metal content of the separated species is obtained from analysis of eluent fractions collected from the outlet of the RI detector. Metal analysis (ICP) is critical since it allows one to determine sample recovery from the column and to obtain accurate data with respect to species distributions. Using RI response data alone for species distribution can be somewhat misleading since RI response for each peak may not be the same.

Separated species can be characterized by elution time (time from injected sample to maximum peak height), elution volume (eluted volume from sample injection to maximum peak height) or Kd, which may be more properly defined as an average Kd value, defined by the equation:

$$Kd = \frac{Ve - Vo}{(Vt - Vo)}$$

where

Ve = elution volume of peak
Vo = exclusion volume of column
Vt = total volume of column

Vo, the exclusion volume of the column, represents a value whereby any species exceeding the molecular weight exclusion limit of the column will elute at Vo. Normally this value is obtained by determining the elution volume of dextran blue (MW $\cong$ 1 million). Vt, the total column volume, is obtained by determining the elution volume of a very low molecular weight material such as HCl. The use of Kd values has the advantage of comprising results obtained from columns of the same type and same eluent but differing somewhat in Vo and Vt values due to the difference in packing and flow rate conditions.

One should keep in mind that Kd values of species refer to separations on a specific GPC resin of specific column length and specific eluent. Thus the same species can have different average Kd values depending upon resin type, eluent system and column length. It should therefore be noted that analyses done on different columns are not directly comparable.

It should be noted that there are no appropriate "marker" compounds available to calibrate the GPC columns used in terms of relating Kd values to specific molecular weights. This is especially true for Zr species. Other workers have estimated molecular weight of Al species found in ACH. These values can be used in a qualitative manner to assign molecular weight ranges of Al species detected by the methods used in carrying out the experiments, the results of which are reflected in the Examples which follow.

Ultracentrifugation Studies (UC)

UC studies of ZAG salts, ZrO(OH) Cl and $ZrOCl_2$ were carried out in order to establish a framework of molecular weight ranges of Zr species as a function of the source of zirconium. For example, Zr polymers in $ZrOCl_2$ were estimated at 25,000 Daltons compared to 2 million for certain ZAG salts (weight average values). These values are considerably larger than the Al polymers in ZAG which are estimated at between 600 - 5000. It is important to note that these measurements were made in bulk solution so that the molecular weight values represent the average of the solution. It can not be used to distinguish between molecular weights of two or more distinct species present in the system.

NMR Studies

Numerous $C_{13}$ and $Al_{27}$ NMR studies were carried out on several ZAG systems, both aqueous and solid state. The results of the work done are important in understanding Zr-glycine and Al-glycine interactions occurring in ZAG systems.

Characterization of ZAG Salts

Aluminum Polymer Distributions in ZAG Salts

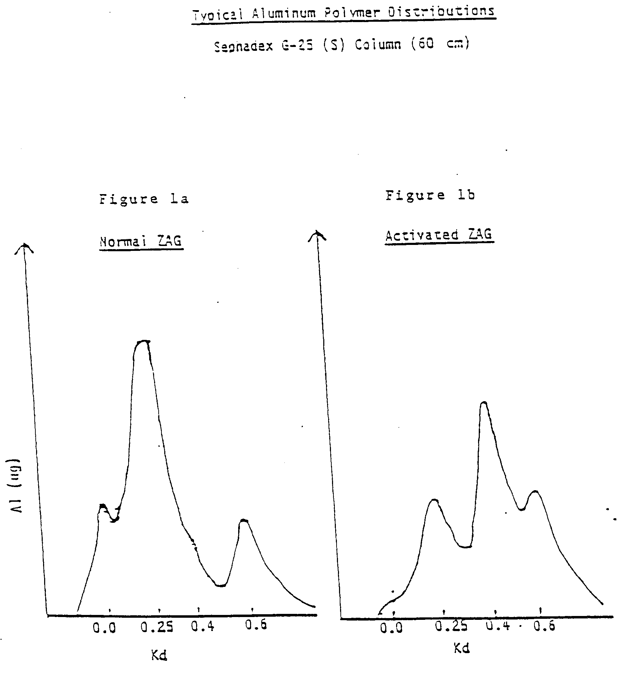

Aluminum polymer distributions in ZAG salts were obtained from GPC-ICP analysis using a Sephadex G-25 (superfine) column (60 cm) and 0.2M KCl/0.0035M HCl eluent. This technique has been used for several years to characterize aluminum containing A/P salts such as aluminum chlorohydrate (ACH). Four distinct aluminum species are generally observed in ACH and in ZAG salts, the distributions of which vary depending upon synthesis routes used to prepare the salts. The Kd values of the four species are 0.0, 0.25, 0.4 and 0.6 with molecular weights ranging from about 5000 Daltons for Kd 0.0 to 600 Daltons for Kd 0.6. As noted previously, the enhanced efficacy of so-called activated ACH salts is attributed to enriched Kd 0.4 content ($\cong$ 50%) for activated ACH compared to about 10% for normal ACH. Similarly, ZAG salts enriched in Al Kd 0.4 are called activated ZAG salts. However, activated ZAG salts may or may not be more efficacious than normal ZAG salts depending upon the corresponding Zr polymer distributions. Figure 1 illustrates typical aluminum polymer distributions in activated and normal ZAG salts, and shows the much larger Al Kd 0.4 peak for the activated ZAG as compared to normal ZAG.

Zirconium Polymer Distributions in ZAG Salts

Characterization of Zr polymers in ZAG salts proved to be a very difficult task due to severe absorption and degradation problems caused by the highly reactive zirconium cationic polymers absorbing and chemically attacking GPC resins. These problems were overcome using Sephacryl resins and 3M KCl/2M glycine eluent.

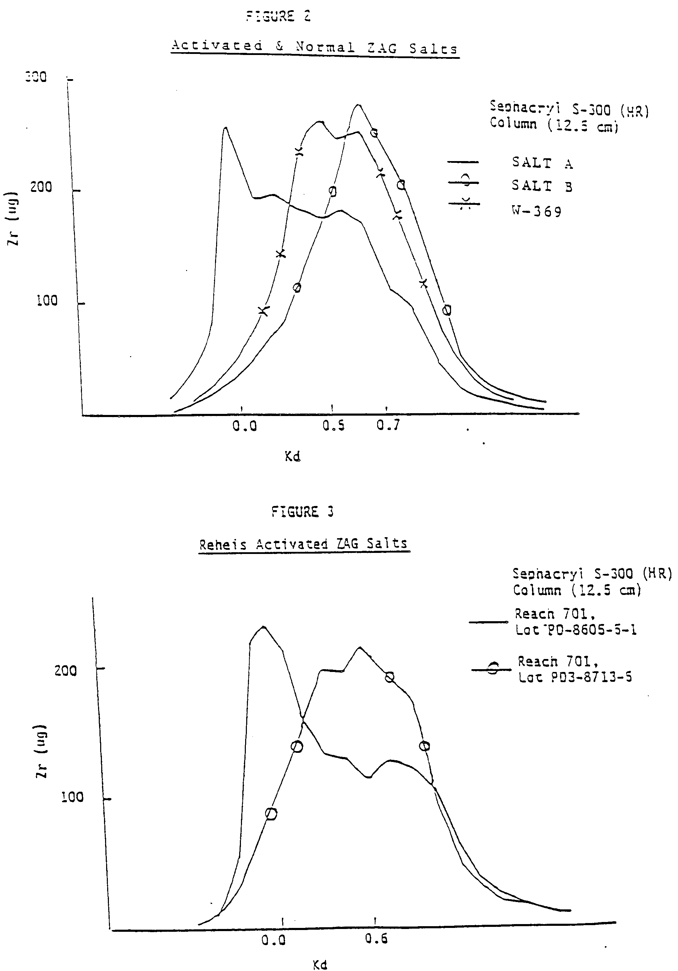

In contrast to Al distributions, where four distinct species are always present in some combination, the Zr species appear to be much larger, are more poly-disperse and can have varying molecular weights depending upon the source material. In some cases only one broad species is observed. Thus, for Zr distributions one must consider the onset of appearance and Kd values when comparing distributions. Since mixed metal Al-Zr species have not been observed, it is assumed that the observed Al and Zr distributions represent that which happens in-vivo. It is speculated that Al-Zr species may exist at very low concentrations in aqueous solution but are unlikely to exist under in-vivo use conditions. Figures 2, 3 and 4 depict Zr polymer distributions for several known activated ZAG salts and a known normal ZAG salt. Zr species of higher molecular weight exhibit lower Kd values and earlier onset of appearance is on the Kd axis. Peak width indicates differences in polydispersity, and can only be confirmed by direct molecular weight measurements such as GPC - Light Scattering.

Figure 2 shows Zr distributions for two lots of a Summit Chemical (formerly Dow-Corning) activated ZAG (SALT A and SALT B) and a normal ZAG salt (W-369) which is commercially available from Summit Chemical. Activated ZAG SALT A represents a production lot with a much higher molecular weight distribution than activated ZAG SALT B, which represents a research lot, as noted by the Kd values of the dominant Zr species (Kd 0.0 for SALT A and Kd 0.65 for SALT B). UC results indicate a molecular weight range of 200,000 - 2 million for SALT A and a weight average of 1.8 million. For W-369 the molecular weight range is estimated at 200,000 to 1 million with a weight average value of 880,000. Again, these values represent the system as a whole and not individual Zr species. Thus, one can see a dramatic change in Zr chemistry resulting from scale-up of SALT B to SALT A. In contrast, the aluminum distribution of SALT A and SALT B are quite similar, as illustrated in Table 2.

As will be seen in the data presented hereinafter, the changes in Zr chemistry results in major consequences with respect to salt efficacy.

Figure 3 shows Zr distributions for two lots of a Reheis activated ZAG salt, designated Reach 701. Again one finds quite different Zr distributions for presumably the same material. The Al distributions were virtually identical.

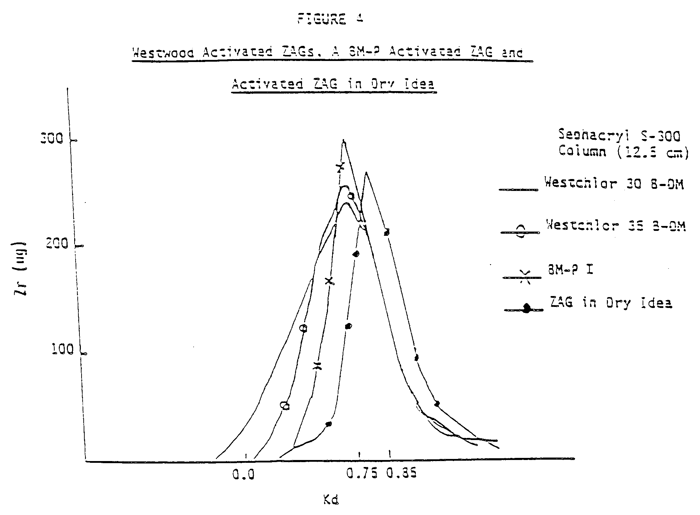

Figure 4 shows Zr distributions for two known Westwood Chemical activated ZAG's, an experimental activated ZAG salt (designated as BM-P I) of the assignee company of this application, and the active component extracted from the commercial consumer product Dry Idea. Here one can easily see that the commercial consumer product has the lowest Zr molecular weight range and is the least polydisperse (narrowest peak) of all the ZAG salts evaluated. Although the Westwood salts have the same Kd value as the BM-P I salt, they have higher molecular weight Zr distributions, as seen by the earlier onset of appearance on the Kd axis.

## Structure - Efficacy Correlations

Once one has developed the ability to characterize ZAG salts with respect to both aluminum and zirconium distributions, it is then possible to determine how these distributions affect salt antiperspirant efficacy. Before looking at specific examples it is instructive for one to be familiar with the historically observed general rank-order efficacy for commonly used antiperspirant salts. This rank-order is shown in Table 1. Included in the table are Al Kd 0.4 content values since these values are used by the industry to distinguish between activated and normal A/P salts. From Table 1, one can see that antiperspirant efficacy increases from normal ACH to activated ZAG. This is the chronological order in which these salts were developed for commercial use. Comparing normal and activated ACH, the increased antiperspirant efficacy of activated ACE is clearly due to increased Al Kd 0.4 content. However, we can also see that a normal ZAG salt is superior to activated ACH, even though the Al Kd 0.4 content of activated ACH is much higher. This indicates that the presence of Zr in A/P salts is more important with respect to overall salt efficacy than any differences in Al species distribution.

The conclusion that Zr species are more efficacious than Al species is consistent with considerations relating to charge/size and hydrolysis properties with respect to diffusion and precipitation of the polymers in the sweat duct.

Finally, one can see that the efficacy of activated ZAG salts can vary, with the best being about 10% drier than normal ZAG.

## Table 1

### Efficacy of Common A/P Salts

| Salt Type | Al Kd 0.4 (% Al) | Relative Efficacy[c] | Estimated Sweat Inhibition[a] (%) |
|---|---|---|---|
| Normal ACH | 5 | --- | 35 |
| Activated ACH | ≥ 50[b] | 15% drier than Normal ACH | 45 |
| Normal ZAG | 15 | 18% drier than Activated ACH | 55 |
| Activated ZAG | ≥ 50[b] | Varies, best are 10% drier than normal ZAG. | 60 |

[a] Based upon historical values and appropriate relative efficacy data.

[b] Threshold level for activated salt set by applicant.

[c] Relative efficacy is related to sweat inhibition by:

$$RE = 1 - \left[ \frac{(100 - \% \text{ Inhibition})_A}{(100 - \% \text{ Inhibition})_B} \right] \times 100$$

where A is the more efficacious of the salt types A and B compared.

Table 2 summarizes the chemical properties of activated ZAG salts in terms of Al Kd 0.4 content, Kd values of the dominant Zr species and onset of appearance of Zr species versus relative efficacy as obtained from clinical efficacy studies where salts are compared head to head. In most cases the salts are tested in the same formulation. This is not the case for the salt BM-P I in BM-P formulations tested against the active in the commercial consumer product even though similar formulation types are used (i.e. solid versus solid, roll-on versus roll-on, etc.) It is believed that differences in the aforementioned formulation other than the A/P salt itself have little or no effect on product efficacy.

The effect of Zr distribution on salt efficacy is most dramatically observed from a comparison of the properties of the activated ZAG SALT A and SALT B and W-369. It is seen that SALT B with a relatively low molecular weight Zr distribution (Kd = 0.65 for the dominant Zr species) is 9.2% drier than W-369, while SALT A with a

8

relative high molecular weight Zr distribution (Kd 0.0 for the dominant Zr species) is 12.6% less effective than W-369, even though both lots have high Al Kd 0.4 content compared to W-369.

Table 2

Structure Efficacy Correlations

| | Chemical Properties | | Efficacy Data | | |
|---|---|---|---|---|---|
| ZAG Salt | Al Kd 0.4 Content (% Al) | Kd Values of Dominant Zr Species [(Sephacryl S-300(HR)] | Control ZAG | Formulation Type | Relative Efficacy |
| W-369 (Control) Summit Chemical | 15 | 0.5 (Figure 2) | -- | -- | -- |
| Commercially Available Product Active (Control) | 57 | 0.85 (Figure 4) | -- | -- | Historically 10% drier than W-369 |
| ZAG SALT B[b] | 60 | 0.65 (Figure 2) | W-369 | Solid | Lot 1 is 9.2% drier than W-369[a] |
| ZAG SALT A[b] | 58 | 0.0 (Figure 2) | W-369 | Non-Aqueous Roll-On | W-369 is 12.6% drier than SALT B |
| Reach 701, (Rehels) | 56 | 0.6 (Figure 3) | W-369 | Solid | 701 is 4% drier than W-369 |
| Westchlor 30-BDM (Westwood) | 53 | 0.75, Very Early onset and very broad (Fig. 4) | Commer. Product Active | Non-Aqueous Roll-On | Control is 10% drier than 30-BDM |
| Westchlor 35-BDM (Westwood) | 43 | 0.75, Early onset and broad (Fig. 4) | Commer. Product Active | Non-Aqueous Roll-On | Control is 10% drier than 35-BDM |
| BM-P I | 53 | 0.75, Later onset than Westchlor salts (Figure 4) | W-369 | Solid | BM-P I is 8.4% drier than W-369[a] |

a = average value of three studies
b = not commercially available

Although Al distributions affect efficacy these results clearly show that differences in Zr distributions is the overriding factor in a given ZAG salts' efficacy. For the above salts, efficacy is in the same rank order as decreasing molecular weight range of the Zr species; SALT B 1 < W-369 < SALT A.

The Reach 701 salt is only 4% more effective than W-369, which is not statistically significant, even though the Al Kd 0.4 content is considerably higher. Here one can see that the Zr molecular weight ranges for both salts are fairly similar with the Reach 701 distribution being somewhat smaller.

Turning to the Westchlor salts, we see that the commercially available product control outperforms Westchlor 30-BDM and 35-BDM by 10%. Although the Westchlor materials have somewhat lower Al Kd 0.4 content, one must look at Zr distributions to fully explain the observed differences in efficacy. Although the Westchlor salts and the active in the commercially available product control have fairly similar Zr Kd values, the Westchlor actives have much larger and broader Zr molecular weight ranges as seen from their earlier onset of appearance along the Kd axis and peak width (see Figure 4).

Finally, one can see that BM-P I is 8.4% drier than W-369.

From the above comparisons it is clear that optimum ZAG efficacy can be achieved by preparing salts having the highest possible Al Rd 0.4 content and the lowest, and least polydisperse molecular weight Zr distributions.

## Synthesis of ZAG Salts of the Present Invention

### General Methodology

The classical method of synthesizing ZAG salts is to react an aluminum component such as ACH or Al $(OH)_3$ with a zirconium component such as $ZrOCl_2$ and/or $ZrO(OH)Cl$ and glycine in one step in the presence of heat and to spray dry the reaction solution to obtain the ZAG salt in a powder form. ZAG solutions are also commercially available for use in A/P products; however, commercial products generally employ only ZAG powder. The OTC monograph for antiperspirants allows several stoichiometric combinations of the above components. The work reflected in the present invention mainly focused on synthesizing so-called tetra ZAG salts using ACH, $ZrOCl_2$, $ZrO(OH)Cl$ and glycine. Tetra ZAG salts can have Al/Zr ratios of 2-6 and Al/Cl ratios of from about 1.5 to 0.9. The amount of glycine used is not restricted but normally is equimolar to the Zr used. The glycine ratio can vary above 0.5. Preferably the glycine ratio will vary between 1 and 3.

While only tetra ZAG salts were used in obtaining the results reflected in the present application, it is expected that other types of ZAG salts can also be used. The Al/Zr ratios and Al/Cl ratios for various types of ZAG salts which can be employed in the present invention are as follows:

| ZAG Type | Al/Zr Ratio | Al/Cl Ratio | Glycine ratio |
|----------|-------------|-------------|---------------|
| TRI | 2 - 6 | 2.1 - 1.5 | Variable above about 0.5 |
| TETRA | 2 - 6 | 1.5 - 0.9 | Variable above about 0.5 |
| PENTA | 6 - 10 | 2.1 - 1.5 | Variable above about 0.5 |
| OCTA | 6 - 10 | 1.5 - 0.9 | Variable above about 0.5 |

### BM-P Type A

This salt had been synthesized prior to the applicants developing structure - efficacy correlations for both Al and Zr distributions. At the time this salt was synthesized, the goal was to maximize Al Kd 0.4 content by a novel process of dry blending activated ACH powder with a $ZrO(OH)Cl-AlCl_3$ -glycine powder to produce a material having the proper stoichiometry for a tetra ZAG salt. Unlike BM-P Type A, the zirconium -glycine solution of present invention does not contain an aluminum species. It has now been determined the Zr molecular weight distribution in BM-P Type A, even when it is prepared in solution (herein designated BM-P I) is fairly low, so that this material outperforms W-369 by only 8.4%.

Knowing how Zr distribution affects efficacy the applicant was in a position to determine how best to synthesize ZAG salts which possess the desired molecular weight distributions, with the synthesis taking place in aqueous solution followed by rapid spray drying to the ZAG salts. Using ZAG powder in antiperspirant products has the advantage of the metal distributions being fixed which may not be the case for ZAG solutions standing over a period of time.

### Factors Affecting Zr Distributions

In the course of applicants' studies it was found that four factors significantly affect the molecular weight range of the Zr species found in the final ZAG product. They are:

1. Source of the Zr starting material
2. Heat
3. Presence and order of addition of glycine
4. Contact time with the Al component in the reaction solution before spray drying.

### Source of Zr Starting Materials

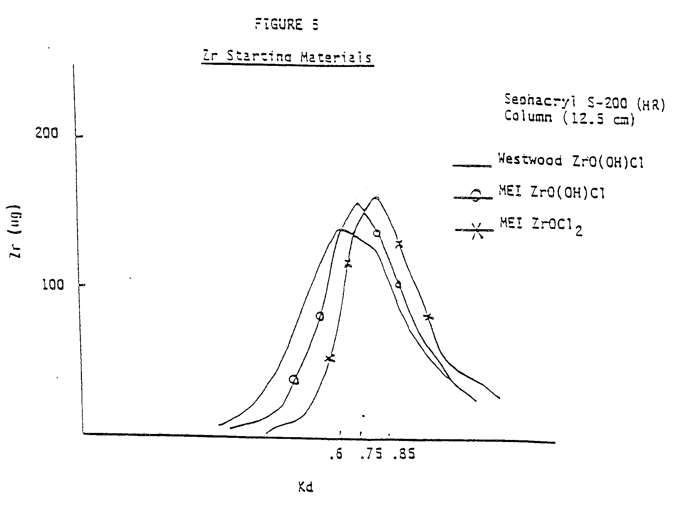

Magnesium Elektron Inc. (MEI) is a primary supplier of $ZrOCl_2$ and $ZrO(OH)Cl$ to antiperspirant salt manufacturers. MEI also supplies Zr carbonate which is used as a starting material to synthesize $ZrO(OH)Cl$ and $ZrOCl_2$ by reacting Zr carbonate paste with HCl. Westwood Chemical Co. also sells $ZrO(OH)Cl$ and uses it to synthesize their own ZAG salts. Westwood buys Zr carbonate paste from MEI to make $ZrO(OH)Cl$. GPC-ICP analysis (Sephacryl S-200 (HR) column) shows the molecular weight range of Zr species in the above materials to be in the relative order of: Westwood $ZrO(OH)C1$ > MEI $ZrO(OH)$ Cl > MET $ZrOCl_2$. Figure 5 illustrates this observation. This is consistent with estimated molecular weight ranges of 40,000 - 400,000, 40,000 - 200,000 and 1000 - 25,000 respectively from UC studies (see Table 3). Since the molecular weight range of Zr species does not decrease during ZAG synthesis, the lowest range attainable is that present in the Zr starting material. Thus the commercially available MEI materials are currently the best known to the inventor to use for the purposes of the present invention based upon its lower molecular weight range.

TABLE 3

| Source | Wgt. Avg. (MW) | MW Distrib. (MW) | Kd Values (200-S Column) |
|---|---|---|---|
| $ZrOCl_2$ Soln (MEI) | 24,800 | 1,000-25,000 | 0.80 |
| $ZrO(OH)Cl$ Soln (MEI) | 180,000 | 40,000-200,000 | 0.72 |
| $ZrO(OH)Cl$ (Westwood) | 350,000 | 40,000-400,000 | 0.60 |

### Application of Heat

The patent literature teaches heating the Al and Zr components of the ZAG complex during synthesis a common practice in the art. In fact, activated ACH is normally prepared by heating aqueous solutions of ACH, the extent of activation, that is to say increased Al Kd 0.4 content being dependent upon ACH concentration, reaction temperature and reaction time. Although heating is good for ACH it has been found to be bad for the Zr component of the system since heat results in Zr polymerization to larger molecular weight species which is contra indicated in the present invention.

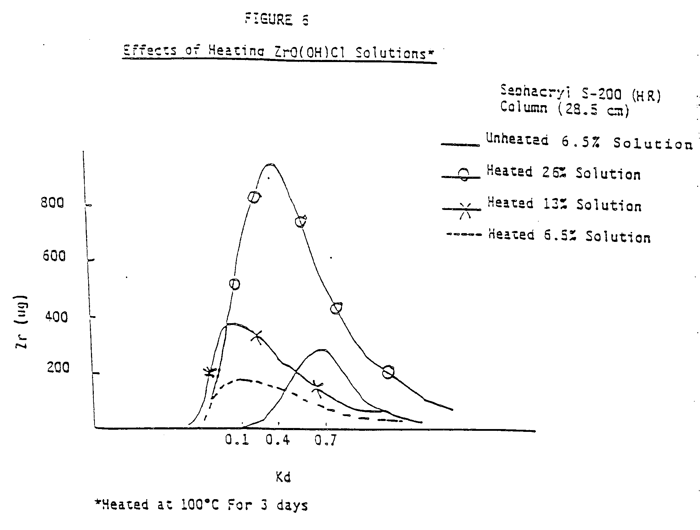

Figure 6 illustrates the effect of heating $ZrO(OH)Cl$ solutions at 100°C for three days. Heated samples show significant polymerization to larger molecular weight Zr species with the effect being more pronounced for the less concentrated solutions. The peak sizes in Figure 6 reflect the difference in sample concentration. While it is true that a 100°C - 3 day heating step is unlikely in commercial synthesis, nevertheless these results show that heat should be avoided for Zr solutions during synthesis. Also there is evidence that $ZrO(OH)Cl$ solution standing at room temperature for about 3 months may have slightly higher molecular weight Zr distributions than when first evaluated. For this reason it is believed that Zr components should be used preferably at room temperature and preferably at the highest possible concentration (26% for MEI $ZrO(OH)Cl$) in order to keep the Zr molecular weight distribution range as small as possible.

### Presence of Glycine

Historically, glycine was added to Al-Zr salts to prevent gelling in aqueous solution and to buffer the acidic salt (glycine increases pH by 0.1 - 0.2 pH units.) More recently, IR, Raman and NMR studies have demonstrated that glycine forms a strong complex with Zr:

$$Zr^+ -- \ ^-OOCCH_2NH_3^+$$

Al - glycine binding does not occur to any significant extent. About 50 - 75% of glycine is bound to Zr in aqueous solution and 100% is bound to Zr in the solid state. The unique ability of glycine to bind to Zr results in glycine acting as an inhibitor for Zr polymerizing to larger species.

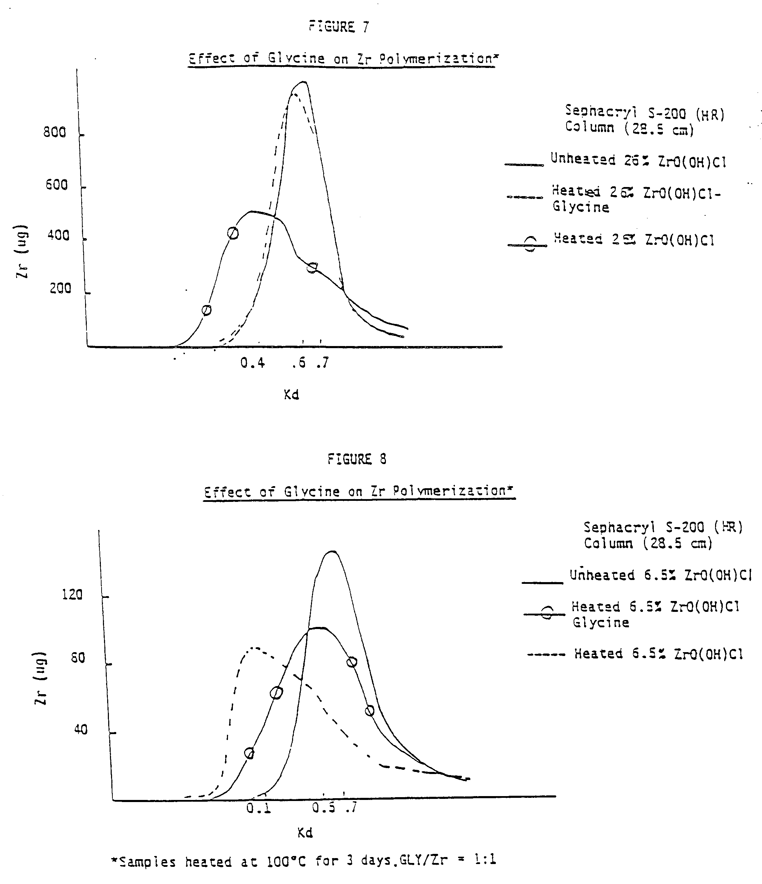

Figures 7 & 8 show the effect of heating ZrO(OH)Cl with an equimolar ratio, and without glycine present. Samples with glycine are much less polymerized by heat than samples not containing glycine. This result indicates that glycine should be added to the Zr component as soon as possible. Ideally, glycine should be added to the Zr starting material immediately after the starting material is itself synthesized in order to minimize any Zr polymerization that may occur upon standing at room temperature.

Contact Time with the Al Component

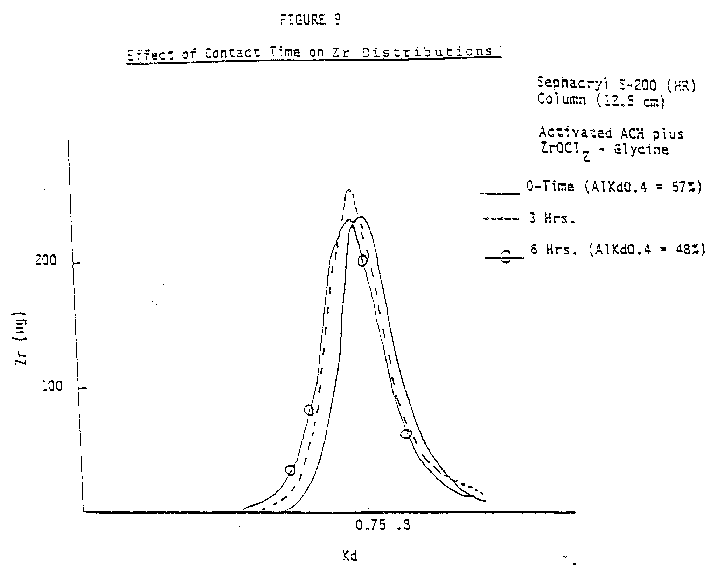

Adding the Al component such as activated ACH to the Zr - glycine component will result in decreased Al Kd 0.4 content and increased molecular weight of the Zr species, the extent of this phenomena being dependent upon contact time and temperature. Figure 9 shows the effect of contact time at room temperature for the synthesis of a laboratory batch of an activated ZAG of the present invention (BM-P II) which contains $ZrOCl_2$ as the Zr component. This batch of BM-P activated ZAG was prepared using 2 moles of ACH/l mole of zirconium oxychloride in glycine. The mole ratio of zirconium/oxychloride was 1/1. The mixture was prepared without the addition of heat and spray dried immediately after mixture. Additional samples were prepared by spray drying after 3 hours and after 6 hours. The results shown in Figure 9 indicate that minimum solution contact time between the Al & Zr component is desirable to maintain Al and Zr distributions present in the starting materials.

Processing Parameters

Based upon the foregoing findings and considerations, it has been found that the preferred ZAG salt complexes of the present invention can be achieved by preparing said ZAG salts using a combination of the following processing parameters:

The Zr -glycine solution must be prepared at ambient temperature, without the application of additional heat beyond that necessary to maintain such ambient temperature. Similarly, the Al and Zr -glycine solutions must be mixed without the application of heat, at ambient temperature.

By ambient temperature, applicants intend to convey the meaning that these steps will be carried at what would normally be considered to be room temperature, that is to say at a temperature of up to about 100°F.

More preferably, the ambient temperature will be from about 65° to about 85°F, and most preferably the ambient temperature will be from about 65° to about 75°F.

The initial reaction, which takes place in forming the ZAG salt complex is endothermic in nature, so that in those circumstances where the "ambient temperature may be too low, due to seasonal variations or variable room temperature conditions, it may on occasion be necessary to add a small amount of heat as necessary to maintain the reaction conditions within the foregoing temperature ranges.

Otherwise, the absence of heat is required and no additional heat should be added as it will serve to reduce the overall desired effectiveness of the resultant ZAG salt complex.

Short residence (contact) times are essential in handling the final mixed ZAG reaction solution prior to forming the ZAG salt in powder form. Ideally, a processing system involving minimal, or essentially zero, residence time will be utilized.

A continuous or batch/continuous operation involving a minimum residence time or contact time for the mixed ZAG reaction solution is preferred, since applicants experiments show that the longer the contact time at this stage of the processing, the worse the resulting ZAG salt will be in terms of its antiperspirant activity.

The choice of processing equipment will dictate the total contact time for the mixed ZAG reaction solution. For this reason, the most preferred processing system will involve the use of a continuous tubular mixing and feeding device to mix the final ZAG reaction components and immediately deliver the mixed reaction solution to a spray dryer in the shortest possible time. While other drying methods may be employed, spray drying is the most rapid method of drying and provides the least exposure of the ZAG salt to heat. Accordingly, spray drying is especially preferred.

Residence or contact times of up to 1 hour, while not desirable, have been found to be acceptable, with residence times of under 30 minutes being desirable. Residence times under about 10 minutes are preferred and an instantaneous tubular flow mixing/spray drying system, having essentially zero residence time, is most preferred.

The foregoing more general discussion of the present invention will be further illustrated by the following specific examples:

EXAMPLES

EXAMPLE I

Laboratory Synthesis of BM-P Activated ZAG Salts

Several ZAG salts of varying Zr starting materials, Al/Zr and Al/Cl ratios were synthesized in the laboratory using an APV lab dryer to obtain the final ZAG powder (60 - 100g batches). Al and Zr component solutions were prepared with no heat and the Zr component was added to and mixed with the Al component with no heat. Reaction solutions were completely spray dried batchwise within 20 - 25 minutes after mixing of components except in cases where long standing times (3 - 6 hours) were chosen to determine their effect on species distribution. The synthesized ZAG salts exhibited high Al Kd 0.4 content ($\cong$ 50%) and Zr distributions similar to those found in the Zr starting materials when spray dried within 20 - 25 minutes of mixing the component solutions. In one case a salt was prepared by pumping both components into a common flow tube and then into the spray dryer (30 second mixing time for components). Similar Al and Zr distributions were observed for this salt and one prepared by mixing the components in a beaker and then spray drying for 22 minutes to dry the batch.

Although a 20 - 25 minute per batch spray drying time produced good results with respect to Al and Zr distributions, synthesizing production quantities of ZAG powder may require much longer spray drying times. Thus it is clear that a properly designed flow system with minimum contact time for the two reaction components is most desirable.

EXAMPLE II

Commercial Synthesis of BM-P Activated ZAG Salts

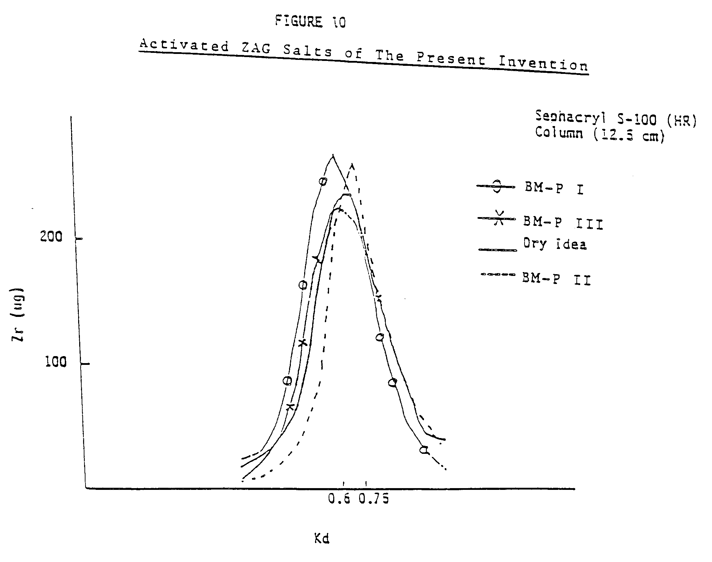

Two BM-P activated ZAG salts, designated BM-P II and BM-P III, were synthesized at APV Crepaco Inc., Attleboro Falls, MA (APV manufacturers spray drying equipment). Al components (I) were prepared in a 50 gallon stainless steel vat while Zr components (II) were prepared in 10 gallon plastic drums at ambient temperature. No heat was used to prepare the components or when II was added to I in the steel vat. The final reaction solution present in the vat was pumped into a commercial spray dryer at a rate of approximately 27 gallons/hr. Each batch (about 10 gallons) was spray dried within 20 - 25 minutes. Two batches of each salt were synthesized, spray dried and collected in the same container. Steam injected into the atomizer was used to densify the spray dried ZAG powder. Table 4 and Figure 10 summarizes

synthesis, processing and chemical data for the BM-P I control and the two ZAG salts prepared in accordance with the present invention.

## Table 4

### Commercially Synthesized BM-P Activated ZAG Salts

| Salt I.D. | Component I (lbs.) | | Component II (lbs.) | | Al Kd 0.4[a] (% Al) | Kd Values of Zr Species[b] | Measured Ratios[f] | | | |
|---|---|---|---|---|---|---|---|---|---|---|
| | | | | | | | $\frac{Al}{Zr}$ | $\frac{Al}{Cl}$ | $\frac{Al+Zr}{Cl}$ | $\frac{Gly^e}{Zr}$ |
| BM-P I | Reach 101 | 9.5 | ZrO(OH)Cl[c] | 17 | 53 | 0.6 (Fig.10) | 3.3 | 0.97 | 1.26 | 1.0 |
| | $H_2O$ | 54.0 | AlCl$_3$ Soln. | 13 | | | | | | |
| | | | Glycine | 2 | | | | | | |
| BM-P II* | Reach 101 | 8.5 | ZrOCl$_2$[d] | 12.4 | 56 | 0.75 (Fig.10) | 3.7 | 0.96 | 1.22 | 1.0 |
| | $H_2O$ | 56.4 | Glycine | 1.5 | | | | | | |
| BM-P III* | Reach 101 | 8.5 | ZrOCl$_2$ | 6.8 | 57 | 0.725 (Fig.10) | 3.4 | 1.02 | 1.32 | 1.0 |
| | $H_2O$ | 56.5 | ZrO(OH)Cl | 7.0 | | | | | | |
| | | | Glycine | 1.7 | | | | | | |
| Active In Dry Idea | --- | | --- | | 57 | 0.65 (Fig.10) | 3.6 | | | |

a - Sephadex G-25 Superfine Column; 60cm
b - Sephacryl S-100 (HR) Column; 12.5 cm
c - MEI ZrO(OH)Cl Soln (26% w/w)
d - MEI ZrOCl$_2$ Soln (32% w/w)
e - Theoretical Values
f - Measured atomic ratios
Spray Dryer - #67 Anhydro flat bottom with sweeper; atomizer speed 19,700 rmp, inlet temp 200°C, outlet temp 90°C, steam pressure 1.5 - 2.0" on Bar gauge, feed rate at 27 gallon/hr.

*Invention

14

As can be seen from Table 4, all BM-P synthesized salts of the present invention have high Al Kd 0.4 content ($\geqq$ 53%) and low molecular weight Zr distributions as noted by the Zr Kd values. From Figure 10 one can see that Zr distributions are in the expected order, taking into consideration the Zr starting material used to synthesize the salts. BM-P II shows the lowest molecular weight Zr distribution and is somewhat lower than the commercial active. BM-P I has the highest molecular weight Zr distributions as evidenced by the significantly lower Kd value. The chemical properties of the salts prepared on a scaled up basis are in fact quite similar to the same salts synthesized in the laboratory.

Thus, applicant was successful in scaling up the synthesis from laboratory to commercial equipment. Moisture loss measurements conducted by APV Crepaco (Cenco balance) on synthesized materials show similar results as commercial ZAG salts such as Westchlor 35 BDM. Thus, spray drying conditions are likely to be close to that used by A/P suppliers. The ZAG salts were milled at Napp Chemical Co., Lodi, N.J. in a jet mill to match the particle size distribution of Wickenol 370 which is currently used in at least one commercial consumer solid antiperspirant product.

## EXAMPLE III

Efficacy Studies - BM-P Activated ZAG Salts

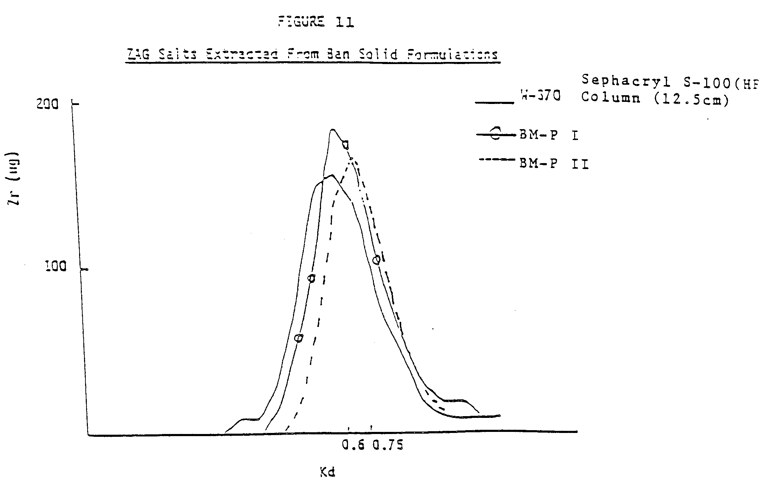

BM-P I, BM-P II and W-370 were formulated into solid low residue formulations. Four month stability at 32°F, RT and 104°F was deemed satisfactory for the above solid sticks. Al and Zr distributions of BM-P I and BM-P II extracted from the sticks were virtually identical to those found in the free salt. This is shown in Figure 11 (extracted BM-P I, BM-P II and W-370). Thus, laboratory processing of solid sticks did not appear to affect the chemical properties of the incorporated ZAG salt. This result is expected to also be true for production processing assuming the processing procedure is fairly similar to the lab process employed.

An efficacy study comparing BM-P I, BM-P II and W-370 in a Solid antiperspirant formulation was conducted at Hilltop Laboratories. The results of the study are shown in Table 5.

Table 5

| Product | Active | Relative Efficacy (1 Hr.) | Statistical Significance | Relative Efficacy (24 Hrs.) | Statistical Significance |
|---|---|---|---|---|---|
| F 5304X (Control) | W-370 | | | | |
| BO 4010-89 | BM-P I | 3% Drier than Control | None | 11% Drier than Control | Yes (p = 0.044) |
| BO 4010-90 | BM-P II[*] | 9% Drier than Control | Yes (p = 0.017) | 13% Drier than Control | Yes (p = 0.017) |

[*]BM-P Activated ZAG Salt of the present invention

The above results clearly indicate that the activated ZAG salts prepared using the novel process of the present invention are more efficacious than the ZAG salt currently used in commercial solid consumer antiperspirant products. The most active salt is BM-P II, whose performance exceeded the anticipated 8 - 10% advantage over W-370 (24 hr. test). The observed rank-order efficacy is consistent with applicant's structure-activity correlations which show that efficacy is enhanced when ZAG salts contain large amounts of the Al Kd

16

0.4 species and relatively low molecular weight Zr species. Al Kd 0.4 content for BM-P II, BM-P I, and W-370 are 57%, 53% and 10% respectively. Figure 12 shows that BM-P II has the lowest molecular weight Zr distribution as compared to BM-P I and W-370.

EXAMPLE IV

Efficacy Study - Invention versus Commercial Product

A clinical efficacy study comparing BM-P II in a solid A/P formulation versus a commercial highly efficacious solid (Dry Idea) is shown in Table 6. As seen from the data reflected in this Table the solid containing BM-P II outperformed Dry Idea by 7% (24 hour test). This advantage is attributed to the molecular weight Zr distribution for BM-P II (Kd = 0.75) compared to the active in Dry Idea (Kd = 0.65).

TABLE 6

| Product | Active | Relative Efficacy 24 Hr. Testing | Statistical Significance |
|---|---|---|---|
| Dry Idea | Control | | |
| BO 4010-90 | BM-P II | 7% Drier than Control | Yes (p = 0.022) |

While a limited number of preferred embodiments of the present invention have been described and tested above, one skilled in the art will, nevertheless, recognize numerous substitutions, modifications and alterations which can be made without departing from the spirit and scope of the invention as limited by the following claims.

## Claims

1. A process for the preparation of an aluminum/glycine/zirconium (ZAG) complex possessing superior antiperspirant characteristics comprising:
   forming an aqueous admixture of a zirconium starting material and glycine at ambient temperature;
   combining said zirconium/glycine aqueous mixture with an aluminum chlorohydrate starting material; and
   rapidly drying the resultant aluminum chlorohydrate/glycine/zirconium mixture.

2. A process according to claim 1, which is carried out at a temperature of up to about 38°C (100°F).

3. A process according to claim 1, which is carried out at a temperature of from about 18°C-30°C (65°F-85°F).

4. A process according to claim 1, which is carried out at a temperature of from about 18°C-24°C (65°F-75°F).

5. A process according to any one of claims 1 to 4, wherein the zirconium/glycine aqueous mixture is prepared without the addition of heat beyond that necessary to maintain said ambient temperature.

6. A process according to any preceding claim, wherein the Zr starting material is selected from $ZrO(OH)Cl$, $ZrOCl_2$ and combinations of both.

7. A process according to any preceding claim, wherein the Zr starting material has a low molecular weight distribution.

8. A process according to any preceding claim, wherein the Zr starting material has a molecular weight distribution of from about 1,000 to about 400,000, as measured by GPC-ICP analysis.

9. A process according to claim 8, wherein the Zr starting material has a molecular weight distribution of from about 1,000 to about 25,000, as measured by GPC-ICP analysis.

10. A process according to claim 8, wherein the Zr starting material has a molecular weight distribution of from about 40,000-200,000, as measured by GPC-ICP analysis.

11. A process according to any preceding claim, wherein the aluminum starting material has an Al Kd 0.4 content of at least 50%.

12. A process according to any preceding claim, wherein the mixed glycine/zirconium is admixed with the aluminum chlorohydrate starting material immediately prior to spray drying in a continuous or semi-continuous operation.

13. An aluminum/glycine/zirconium complex obtained by a process as claimed in any one of claims 1 to 12.

14. An aluminum/glycine/zirconium complex having superior antiperspirant activity comprising the admixture product of an aluminum chlorohydrate salt, a zirconium starting material and glycine, wherein the complex exhibits a Zr polymer molecular weight distribution of from about 1,000 to about 200,000, as measured by GPC-ICP analysis.

15. The aluminum/glycine/zirconium complex according to claim 14, which exhibits a Zr polymer molecular weight distribution of from about 1,000 to about 25,000, as measured by GPC-ICP analysis.

16. The aluminum/glycine/zirconium complex according to claim 14, which exhibits a Zr polymer molecular weight distribution of from about 40,000 to about 200,000 as measured by GPC-ICP analysis.

17. The aluminum/glycine/zirconium complex according to any one of claims 14 to 16, having a Zr Kd of greater than about 0.65 as determined using a Sephacryl S-100 (HR) Column (12.5cm).

18. The aluminum/glycine/zirconium complex according to claim 17, having a Zr Kd of not less than about 0.725, as determined using a Sephacryl S-100 (HR) Column (12.5cm).

19. A non-aqueous antiperspirant composition comprising, as at least one active antiperspirant ingredient, an aluminum/glycine/zirconium complex as claimed in any one of claims 14 to 18 or obtained by a process as claimed in any one of claims 1 to 12.

20. A non-aqueous antiperspirant composition as claimed in claim 19 in a cosmetically acceptable carrier or vehicle.

Typical Aluminum Polymer Distributions

Sephadex G-25 (S) Column (60 cm)

Figure 1a

Normal ZAG

Figure 1b

Activated ZAG

FIGURE 2

Activated & Normal ZAG Salts

FIGURE 3

Reheis Activated ZAG Salts

FIGURE 4

Westwood Activated ZAGs, A 8M-P Activated ZAG and

Activated ZAG in Dry Idea

FIGURE 5

Zr Starting Materials

Sephacryl S-200 (HR)
Column (12.5 cm)

——— Westwood ZrO(OH)Cl

—○— MEI ZrO(OH)Cl

—✕— MEI ZrOCl₂

FIGURE 6

Effects of Heating ZrO(OH)Cl Solutions*

Sephacryl S-200 (HR)
Column (28.5 cm)

_____ Unheated 6.5% Solution

__O__ Heated 26% Solution

__X__ Heated 13% Solution

----- Heated 6.5% Solution

*Heated at 100°C For 3 days

FIGURE 7

Effect of Glycine on Zr Polymerization*

Sephacryl S-200 (HR)
Column (28.5 cm)

———— Unheated 26% ZrO(OH)Cl

- - - - Heated 26% ZrO(OH)Cl-
Glycine

—⊖— Heated 26% ZrO(OH)Cl

FIGURE 8

Effect of Glycine on Zr Polymerization*

Sephacryl S-200 (HR)
Column (28.5 cm)

———— Unheated 6.5% ZrO(OH)Cl

—⊖— Heated 6.5% ZrO(OH)Cl
Glycine

- - - - Heated 6.5% ZrO(OH)Cl

*Samples heated at 100°C for 3 days. GLY/Zr = 1:1

24

FIGURE 9

Effect of Contact Time on Zr Distributions

Sephacryl S-200 (HR)
Column (12.5 cm)

Activated ACH plus
$ZrOCl_2$ - Glycine

———— 0-Time (AlKd0.4 = 57%)

------ 3 Hrs.

——O—— 6 Hrs. (AlKd0.4 = 48%)

FIGURE 10

Activated ZAG Salts of The Present Invention

FIGURE 11

ZAG Salts Extracted From Ban Solid Formulations

European Patent Office

**EUROPEAN SEARCH REPORT**

Application Number

EP 94 30 5785

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP-A-0 499 456 (BRISTOL-MYERS SQUIBB COMPANY) <br> * claim 15 * | 1 | A61K7/38 <br> C07F7/00 |
| Y | US-A-5 225 187 (W.J. CARMODY) <br> * claim 3 * | 1 | |
| A | DE-A-26 05 386 (ARMOUR PHARMACEUTICAL CO.,) <br> * example 14 * | 1 | |
| A | EP-A-0 393 275 (WESTWOOD CHEMICAL CORPORATION) <br> * claims 8-11 * | 1 | |
| | | | **TECHNICAL FIELDS SEARCHED (Int.Cl.6)** |
| | | | A61K <br> C07F |

The present search report has been drawn up for all claims

| Place of search | Date of completion of the search | Examiner |
|---|---|---|
| BERLIN | 11 October 1994 | Kapteyn, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)